# EUROPÄISCHE PATENTSCHRIFT

(11) **EP 0 937 698 B1**
(45) Veröffentlichungstag und Bekanntmachung des Hinweises auf die Patenterteilung: **02.05.2003**
(21) Anmeldenummer: 99102932.3
(22) Anmeldetag: 13.02.1999
(51) Int. Cl.: C07C 29/149, C07C 31/02, C07C 31/13, B01J 21/06

(54) **Verfahren zur Herstellung von Alkoholen**
Process for the production of alcohols
Procédé pour la préparation d'alcools

(30) Priorität: 20.02.1998 DE 19807268
(43) Veröffentlichungstag der Anmeldung: 25.08.1999
(73) Patentinhaber: BASF AKTIENGESELLSCHAFT, 67056 Ludwigshafen (DE)
(72) Erfinder: Merger, Martin, Dr., 67227 Frankenthal (DE); Liang, Shelue, Dr., 67071 Ludwigshafen (DE); Fischer, Rolf, Dr., 69121 Heidelberg (DE); Wulff-Döring, Joachim, Dr., 67227 Frankenthal (DE)

(56) Entgegenhaltungen:
- EP-A- 0 585 053
- DE-A- 4 021 230
- US-A- 5 008 235
- DATABASE WPI Section Ch, Week 8726 Derwent Publications Ltd., London, GB; Class A41, AN 87-180567 XP002099846 & JP 62 108832 A (MITSUBISHI CHEM IND LTD) , 20. Mai 1987

## Beschreibung

Die Erfindung betrifft ein Verfahren zur Herstellung von Alkoholen, durch Gasphasenhydrierung von Carbonsäuren oder deren Estern bei Temperaturen von 250 bis 400°C und Drücken von 5 bis 100 bar in Gegenwart von überwiegend Oxide der vierten Haupt- und Nebengruppe, insbesondere Zirkondioxid enthaltenden Katalysatoren, in Gegenwart von Alkoholen.

Es ist aus DE-A-2 519 817 bekannt, Carbonsäuren in der Flüssigphase, z. B. in Gegenwart von Katalysatoren, die Rhenium und/oder Elemente der achten Nebengruppe enthalten, zu Alkoholen zu hydrieren. Hierfür werden im allgemeinen hohe Temperaturen von 200°C und mehr und hohe Drucke von 200 bar und darüber benötigt. Die Katalysatoren lassen sich in vielen Fällen nach ihrer Desaktivierung nicht regenerieren. Wenn sie große Mengen an teuren Metallen enthalten, müssen die Katalysatoren aufgearbeitet werden, um die Metalle zurückzugewinnen. Ist eine Aufarbeitung aus technischen Gründen nicht möglich oder aus wirtschaftlicher Sicht nicht angezeigt, müssen die verbrauchten Katalysatoren, meist unter hohem Kostenaufwand, deponiert werden.

Aus DE-A-2 543 673 ist weiterhin bekannt, Carbonsäureester in der Flüssigphase oder Gasphase, vor allem in Gegenwart von überwiegend Kupfer enthaltenden Katalysatoren, wie Kupferchromit-Katalysatoren oder Kupfer, Mangan und Aluminium enthaltenden Katalysatoren, zu Alkoholen zu hydrieren. Bei Ester-Hydrierungen in der Flüssigphase werden im allgemeinen wie im Fall der Carbonsäure-Hydrierung Temperaturen über 200°C und Drucke über 200 bar benötigt (WO 97/31882). Führt man Ester-Hydrierungen in der Gasphase durch, so bleiben die Hydriertemperaturen im Bereich von 200°C, die Reaktionsdrucke sinken dagegen in einen Bereich um 50 bar ab (U.S. 5.395.990).

Kupfer-Katalysatoren besitzen den Nachteil der Empfindlichkeit gegenüber Halogen-Verunreinigungen in den Ausgangsverbindungen und den Nachteil einer begrenzten Katalysator-Standzeit. Eine Regenerierung ist in den meisten Fällen nicht möglich, so daß sich ebenfalls eine Aufarbeitung oder Deponierung der gebrauchten Katalysatoren anschließen muß.

Es bestand daher die Aufgabe, ein Verfahren zur Herstellung von Alkoholen durch Hydrierung von Carbonsäuren und ihren Estern zu entwickeln, das bei möglichst niedrigem Druck betrieben werden kann und dessen Hydrierkatalysatoren regeneriert werden können.

Erste Ansätze hierzu sind aus der Patentliteratur bekannt:

So gelingt es nach EPA 285 786, Carbonsäuren und ihre Ester anstelle von Wasserstoff mit Alkoholen, insbesondere 2 - 6 Kohlenstoffatome enthaltenden Alkoholen als Wasserstoffüberträger zu entsprechenden Alkoholen zu hydrieren. Besonders bevorzugt dabei ist i-Propanol. Die Umsetzung erfolgt bei 250 - 450°C und Normaldruck in der Gas- und Flüssigphase in Gegenwart von nur teilweise dehydratisierten Zirkon-, Titan- oder Zinnoxiden, die z. B. durch Erhitzen entsprechender Hydroxide auf Temperaturen von bis zu 300°C hergestellt werden.

Weiterhin ist in EPA 585 053 beschrieben, Carbonsäuren und ihre Derivate, wie Ester, Nitrite oder Amide, mit Alkoholen als Wasserstoffüberträger in der Gas- oder Flüssigphase in Gegenwart von TiO₂/ZrO₂-Katalysatoren zu entsprechenden Alkoholen umzusetzen. Im Vergleichsbeispiel 1 ist auch die Umsetzung von n-Decansäure zu n-Decanol mit i-Propanol in Gegenwart von nur teilweise dehydratisiertem Zirkonhydroxid beschrieben. Für 300°C und Normaldruck wird eine n-Decanol-Ausbeute von 34 % (Umsatz 100 %) angegeben.

Anstelle von TiO₂/ZrO₂-Katalysatoren sind nach JP 060 651 25 und JP 060 634 01 auch Wismut-oxid/ZrO₂ und Zinnoxid/ZrO₂-Katalysatoren verwendbar.

Die Verwendung von Alkoholen als alleiniger Wasserstofflieferant hat den Nachteil, daß die für die Hydrierung eingesetzten Alkohole in entsprechende Ketone umgewandelt werden und damit verloren gehen. Da pro Mol Carbonsäure oder Ester zwei Mole Alkohol verbraucht werden und das besonders kostengünstige Methanol nach der zitierten Literatur nicht zu den bevorzugten Hydrieralkoholen gehört, ist diese Methode nur für die Herstellung hochpreisiger Alkohole nutzbar.

In JP 6 2108-832-A ist schließlich die Gasphasen-Hydrierung von aliphatischen und cycloaliphatischen Carbonsäuren mit Wasserstoff bei 200 - 500°C und Normaldruck in Gegenwart von bei 300 bis 950°C calciniertem Zirkondioxid, das mit weiteren Elementen wie vor allem Chrom dotiert sein kann, zu entsprechenden Aldehyden und/oder Alkoholen beschrieben.

In allen Patentbeispiele in JP 6 2108-832 wird die Hydrierung mit Wasserstoff bei Normaldruck durchgeführt. Ausgehend von cycloaliphatischen Carbonsäuren wie Cyclohexancarbonsäure wird mit undotiertem Zirkondioxid bei 300 - 350°C und Normaldruck mit Selektivitäten von 98 - 99 % überwiegend Cyclohexancarbaldehyd erhalten (Beispiele 1 - 7). Mit der aliphatischen Carbonsäure Pivalinsäure wird Pivaldehyd mit 100 %iger Selektivität erhalten (Beispiel 8). Ausgehend von aliphatischen Carbonsäuren oder ihren Estern, die zwei alpha-Wasserstoffatome besitzen, werden dagegen mit chromdotiertem Zirkondioxid bei 320 °C und Normaldruck die entsprechenden Alkohole oder Gemische aus Alkoholen und Aldehyden im mäßigen Ausbeuten und Selektivitäten erhalten. So erzielt man ausgehend von n-Heptansäure und n-Heptansäuremethylester n-Heptanol-Ausbeuten von 62 % bzw. 33,9 % (Beispiele 9 und 10), ausgehend von Propionsäure (Beispiel 14) und Valeriansäure (Beispiel 16) n-Propanol mit einer Ausbeute von 34,6 % und n-Pentanol nur in Spuren (n-Valeraldehyd-Ausbeute 37,1 %). Ausgehend von Buttersäure (Beispiel 15) wird ein Gemisch aus n-Butyraldehyd (Ausbeute 15,8 %) und n-Butanol (Ausbeute 30 %) gefunden.

Eine Lehre, wie man ausgehend von aliphatischen und cycloaliphatischen Carbonsäuren und ihren Estern mit hohen Ausbeuten und Selektivitäten entsprechende Alkohole erhält, wird im JP 6 2108-832 A nicht gegeben. Es wird nur angemerkt, daß mehr Alkohol erhalten wird, wenn die Zahl der Kohlenstoffatome in der Carbonsäure niedrig ist. Weiterhin, daß die Alkoholmenge steigt, wenn die Reaktion unter Druck durchgeführt wird.

Es bestand daher insbesondere die Aufgabe, ein Verfahren vorzuschlagen, das es ermöglicht, ohne die Nachteile des vorgenannten Standes der Technik Alkohole in sehr guten Ausbeuten bei mittleren Drücken herzustellen.

Diese Aufgabe wurde erfindungsgemäß gelöst mit einem Verfahren zur Herstellung von Alkoholen durch Gasphasenhydrierung von Carbonsäuren oder Estern bei erhöhter Temperaturen und erhöhtem Druck in Gegenwart von Katalysatoren, die als hydrieraktive Bestandteile Oxide der vierten Hauptgruppe und/oder Nebengruppe enthalten oder aus diesen bestehen, dadurch gekennzeichnet, bei dem man die Hydrierung
a) bei Temperaturen von 250°C bis 400°C und Drücken von 5 bar bis 100 bar in Gegenwart von
b) primären oder sekundären Alkoholen vorzugsweise Methanol durchführt, wobei das Molverhältnis Wasserstoff zu Alkohol maximal 800, vorzugsweise 10 bis 300 und insbesondere 20 bis 200 beträgt.

Es war überraschend, daß es durch die spezielle Maßnahmenkombination gelingt, Carbonsäuren und ihre Ester mit hohen Ausbeuten und Selektivitäten zu den entsprechenden Alkoholen zu hydrieren, wobei weder die hohen Drücke gemäß JP 59106-431 erforderlich sind noch die zugesetzten Alkohole in solchen Mengen dehydriert werden, daß dadurch die Wirtschaftlichkeit beeinträchtige würde.

Als Ausgangsstoffe für das erfindungsgemäße Verfahren können gesättigte aliphatische, cycloaliphatische, aromatische und heterocyclische Mono- und Polycarbonsäuren und ihre Ester eingesetzt werden. Beispiele sind Essigsäure, n-Hexansäure, n-Hexansäuremethylester, n-Decansäure, Valeriansäuredimethylester, Cyclohexancarbonsäure, Cyclohexancarbonsäuremethylester, 1,4-Cyclohexandicarbonsäuredimethylester, Cyclohexancarbonsäure-n-butylester, Terephthalsäure, Terephthalsäuredimethylester, Benzoesäuremethylester, Benzoesäure, Nicotinsäure, Nicotinsäuremethylester, Tetrahydropyran-4-carbonsäure, Tetrahydropyran-4-carbonsäuremethyl ester, Tetrahydrofuran-3-carbonsäuremethylester oder Furan-3-carbonsäuremethylester oder Acrylsäuremethylester.

Verwendet man Ester als Ausgangsverbindungen, so sind Ester mit C₁-C₄-Alkoholkomponenten bevorzugt und mit C₁- und C₂-Alkoholkomponenten besonders bevorzugt.

Als Alkohole kommen primäre und sekundäre aliphatische, cycloaliphatische, aromatische oder heterocyclische Alkohole in Betracht. Sie sollten unter den angegebenen Reaktionsbedingungen gut verdampfbar sein. Bevorzugt sind aliphatische, primäre und/oder sekundäre Alkohole mit einem bis sieben Kohlenstoffatomen. Beispiele hierfür sind Methanol, Ethanol, n-Propanol, i-Propanol, n-Butanol, sec-Butanol, n-Pentanol-, Cyclohexanol, Benzylalkohol. Besonders bevorzugt ist Methanol, das nach EP 585 053 und JP 6 2108-832 in Abwesenheit von Wasserstoff nicht zu den bevorzugten Alkoholen gehört.

Als Katalysatoren kommen Oxide der vierten Haupt- und Nebengruppe, vor allem Titandioxide, Zirkondioxide, Hafniumdioxide und Zinnoxide, in Betracht.

Bevorzugt sind Zirkondioxide, die in der monoklinen, kubischen oder tetragonalen Kristallgitterform vorliegen können. Insbesondere wird Zirkondioxid, zu 50 - 100 %, bevorzugt 60 - 98 %, besonders bevorzugt zu 70 - 95 % aus monoklinem Zirkondioxid besteht verwendet. Diese Katalysatoren sind, sofern nicht käuflich, in an sich bekannter Weise erhältlich (vgl. z. B. Catal. Rev. Sci. Eng. 27, Seite 341 bis Seite 372 (1985)). Sie werden beispielsweise durch Lösen von Zirkonverbindungen, z. B. Zirkonnitrat oder Zirkonylchlorid, in Wasser, Ausfällen von Zirkonhydroxiden mit Alkali-, Erdalkalihydroxiden oder Ammoniak, Waschen des erhaltenen wasserhaltigen Zirkonhydroxids, Trocknen und Calcinieren bei 300 bis 850°C erhalten.

Das verwendete Zirkoniumdioxid hat in der Regel eine BET-Oberfläche von 5 bis 150, vorzugsweise 20 bis 130 und insbesondere 40 bis 120 m²/g, wobei unter der BET-Oberfläche die nach der Brunauer-Emmett-Teller-Methode bestimmte Katalysator-Oberfläche zu verstehen ist (vgl. Z. Anal. Chem. 238, Seite 187 (1968)). Das Zirkondioxid sollte neutral sein, das heißt möglichst wenige basische und saure Zentren enthalten. So können basische Katälysatorzentren z. B. durch Aldolkondensation der bei der Hydrierung durchlaufenen Aldehyde zu Ausbeuteverlusten führen. Saure Katalysatorzentren können die Ausbeuten der gewünschten Alkohole durch Wasserabspaltung zu Olefinen oder durch Etherbildung verringern.

Die ZrO₂-Katalysatoren können als Trägerkatalysatoren auf einem an sich bekannten inerten Träger wie Siliciumdioxid oder Aluminiumoxid und vor allem als Vollkatalysatoren eingesetzt werden. Sie eignen sich in Form von Strängen, vorzugsweise von 1 bis 5 mm größtem Durchmesser, Pellets oder Kugeln. (Alle Mengenangaben über die Zusammensetzung der Katalysatoren beziehen sich auf deren aktive Masse, berücksichtigen also die Trägermaterialien nicht).

Es kann sinnvoll sein, dem Zirkondioxid zur Steigerung der Strukturstabilität und Standzeit des Katalysators geringe Mengen eines oder mehrere Elemente hinzuzufügen. Beispiele für derartige Elemente, die im fertigen Zirkondioxid-Katalysator überwiegend in oxidischer Form vorliegen, sind Lanthanid-Elemente wie Lanthan, Cer, Praseodym, Neodym, Promethium, Samarium, Europium, Gadolinium, Terbium, Dysprosium, Holmium, Erbium, Thulium, Ytterbium und Lutetium.

Vor allem die Verbindungen des Lanthans und Praseodyms, und daneben die der Cers, Samariums, Neodyms und Europiums, eignen sich als Bestandteile der erfindungsgemäß verwendeten Katalysatoren.

Weiterhin sind zur Mitverwendung Elemente wie Chrom, Mangan, Zink, Indium, Zinn, Blei, Vanadium oder Eisen geeignet. Chrom ist vor allem dann angezeigt, wenn aliphatische Carbonsäuren und Ester zu Alkoholen hydriert werden sollen.

Das Atomverhältnis des zugesetzten Elements zu Zirkondioxid beträgt im allgemeinen 0,005 bis 0,3, bevorzugt 0,05 bis 0,2.

Die Katalysatoren können in einem Festbett- oder einem Wirbelbettreaktor angeordnet sein. Die Hydrierung kann von oben nach unten oder von unten nach oben, durchgeführt werden. Dabei wird zweckmäßig mindestens so viel Wasserstoff als Hydriermittel und Trägergas verwendet, daß Edukte, Zwischenprodukte und Produkte während der Reaktion nie flüssig werden. Der überschüssige Wasserstoff und nicht dehydrierter Alkohol werden vorzugsweise im Kreis geführt, wobei ein kleiner Teil als Abgas zur Entfernung von. Inerten wie Methan und Kohlenmonoxid ausgeschleust werden kann. Es können dabei ein Reaktor oder mehrere Reaktoren, hintereinander oder parallel zueinander geschaltet, verwendet werden.

Die Hydriertemperatur beträgt 250°C bis 400°C, bevorzugt 270°C bis 370°C, besonders bevorzugt 300 bis 350°C.

Der Reaktionsdruck bei Verwendung von undotiertem Zirkondioxid beträgt 5 bar bis 100 bar, bevorzugt 10 bar bis 60 bar, besonders bevorzugt 25 bar bis 50 bar. Bei Verwendung von dotiertem Zirkondioxid beträgt der Druck 20 bis 100 bar, bevorzugt 30 bis 50 bar, da die Hydrierung zum Alkohol offenbar durch die Dotierung verlangsamt wird.

Das Molverhältnis von Wasserstoff zur eingesetzten Carbonsäure oder dem Ester beträgt im allgemeinen 20 bis 300, bevorzugt 50 bis 150, besonders bevorzugt 70 bis 130.

Desaktivierte Zirkondioxid-Katalysatoren lassen sich z. B. durch Abbrennen mit Luft bei Temperaturen von 300°C bis 700°C viele Male regenerieren.

Die Menge an Alkohol beträgt im allgemeinen 0,5 bis 10 Mole, bevorzugt 1 bis 5 Mole, besonders bevorzugt 2 bis 4 Mole Alkohol pro Mol eingesetzte Säure bzw. Ester.

Die Katalysator-Belastung beträgt in der Regel 0,01 bis 0,3, bevorzugt 0,05 bis 0,2, besonders bevorzugt 0,05 bis 0,15 kg zu hydrierender Ester oder Säure pro Liter Katalysator/Stunde.

Die Hydrierung wird zweckmäßig kontinuierlich durchgeführt. Die Hydrierausträge werden nach der Kondensation des Hydrieraustrags bevorzugt destillativ aufgearbeitet.

Der Hydrieraustrag besteht im wesentlichen aus dem durch Hydrierung gebildeten Alkohol, weiterhin durch Hydrierung von Ester- oder Säuregruppen freigesetztem Alkohol oder Wasser, zugesetztem Alkohol und nicht umgesetzter Säure, Ester oder intermediär auftretendem Aldehyd. Als Nebenprodukte können Olefine, Kohlenwasserstoffe oder Ether auftreten. Durch Veresterung oder Umesterung können neue Ester gebildet werden, die jedoch zu den gewünschten Alkoholen hydrierbar sind.

Überschüssiger zugesetzter Alkohol und bei unvollständigem Umsatz im Hydrieraustrag enthaltene Ausgangs- und Zwischenverbindungen können destillativ abgetrennt und in die Hydrierstufe zurückgeführt werden.

### Beispiele

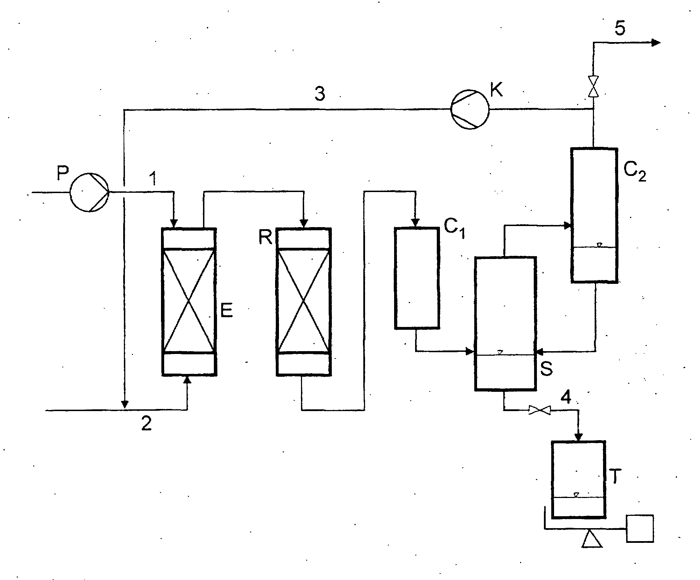

a) Hydrierapparatur:
   Die Versuche wurden in der in Fig. 1 schematisch dargestellten Hydrierapparatur kontinuierlich durchgeführt. Sie besteht aus einem Verdampfer (E), einem 1,4 l-Rohrreaktor (30 x 2000 mm) (R), zwei Kühlern C₁ und C₂ und einem Druckabscheider (S) zur Gewinnung der kondensierbaren Komponenten aus dem Wasserstoffstrom, einem Kreisgasverdichter (K) zur Rückführung des Kreisgaswasserstoffs (3) und einem Austragsgefäß (T) zum Sammeln des Reaktionsaustrags (4).
b) Durchführung der Versuche:
   In den Beispielen 1 bis 7, 9, 11 und 12 wurde Zirkondioxid der Firma Norton verwendet, mit der Bezeichnung XZ 16075. Hierbei handelt es sich um 3 mm Stränge mit einer BET-Oberfläche um 50m²/g. Das Zirkondioxid war zu mehr als 90 % monoklin.

In den Beispielen 8 und 10 wurde La₂O₃ (3 % La)/Zirkondioxid verwendet.

Zur Herstellung von La₂O₃ (3 % La₂O₃)/ZrO₂ wurden 3 mm ZrO₂-Stränge aus ZrO₂ der Firma Norton (SN 9516321) mit La (NO₃)₃-Lösung getränkt, 4 Stunden bei 120°C getrocknet und 6 Stunden bei 400°C calciniert.

In Beispiel 14 wurde Cr₂O₃ (5 % Cr)/Zirkondioxid verwendet.

Zur Herstellung von Cr₂O₃ (5 % Cr)/ZrO₂ wurden 3 mm ZrO₂-Stränge aus ZrO₂ der Firma Norton (SN 9516321) mit Cr(NO₃)₃-Lösung getränkt, 4 Stunden bei 120°C getrocknet und 6 Stunden bei 400°C calciniert.

Die Katalysatorzone wurde am oberen und unteren Ende durch eine Schicht Quarzringe begrenzt. In allen Fällen wurden mit Kreisgas gearbeitet, wobei 10 % des Kreisgases (5) ausgeschleust und durch die gleiche Menge Frischwasserstoff ersetzt wurden.

Die Ausgangsverbindungen (1), (7), (12) und zugesetzter Alkohol wurden mit einer Pumpe (P) in den Verdampfer dosiert, wo sie verdampft und mit vorgeheiztem Wasserstoff (2) vermischt gasförmig in den Reaktor geleitet wurden. Das Molverhältnis Ausgangsverbindung/Wasserstoff der Eingangsströme 1 und 2 wurde durch Wägung der zugeführten Menge an Ausgangsprodukt und Messung der Wasserstoffströme ermittelt.

Der Hydrieraustrag wurde nach dem Kondensieren gewogen. Seine Zusammensetzung wurde gaschromatographisch unter Verwendung eines inneren Standards (Diethylenglykoldimethylether) quantitativ bestimmt. Jede Versuchseinstellung wurde mehrere Tage ohne Änderung betrieben, erst dann wurde der Hydrieraustrag analysiert.

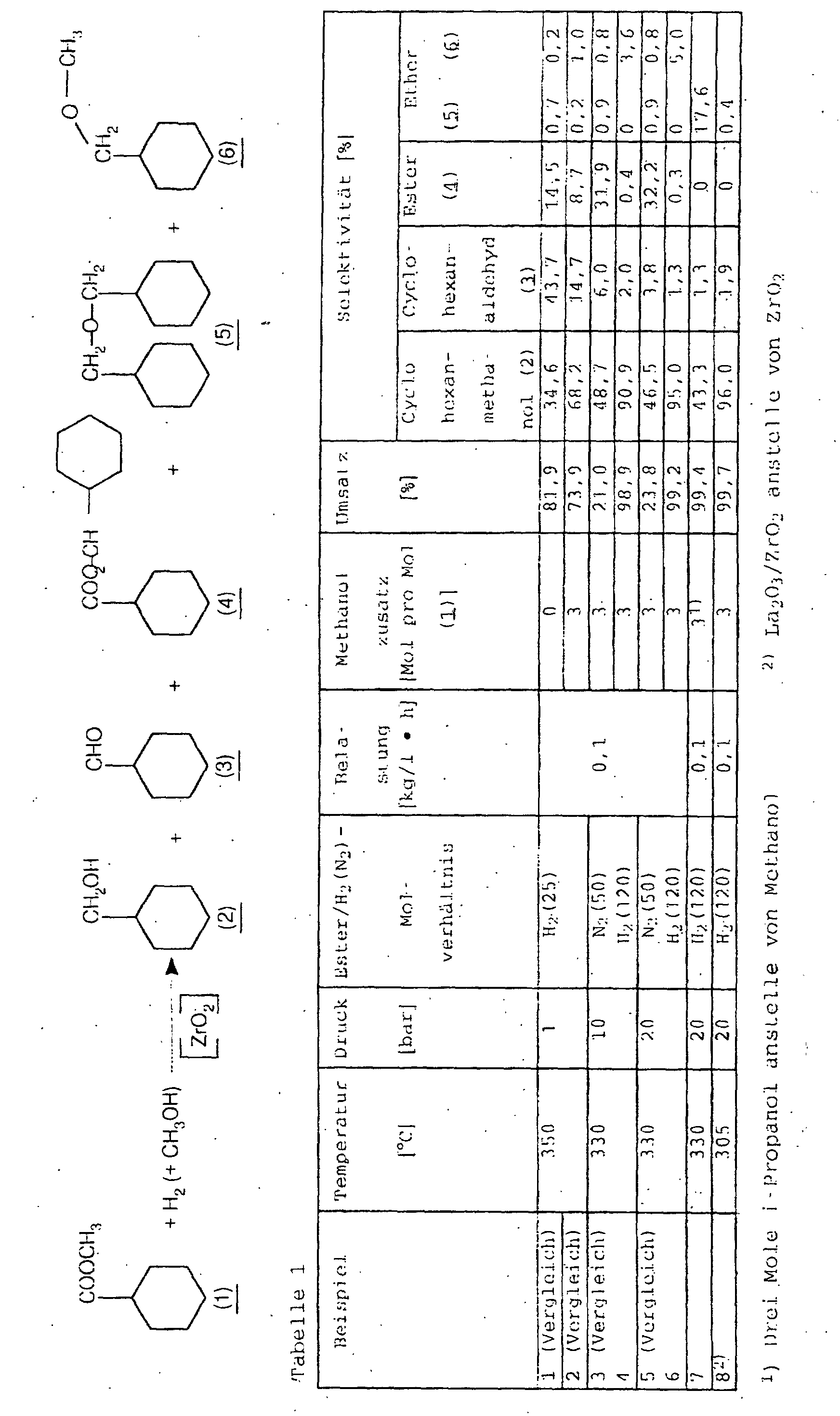

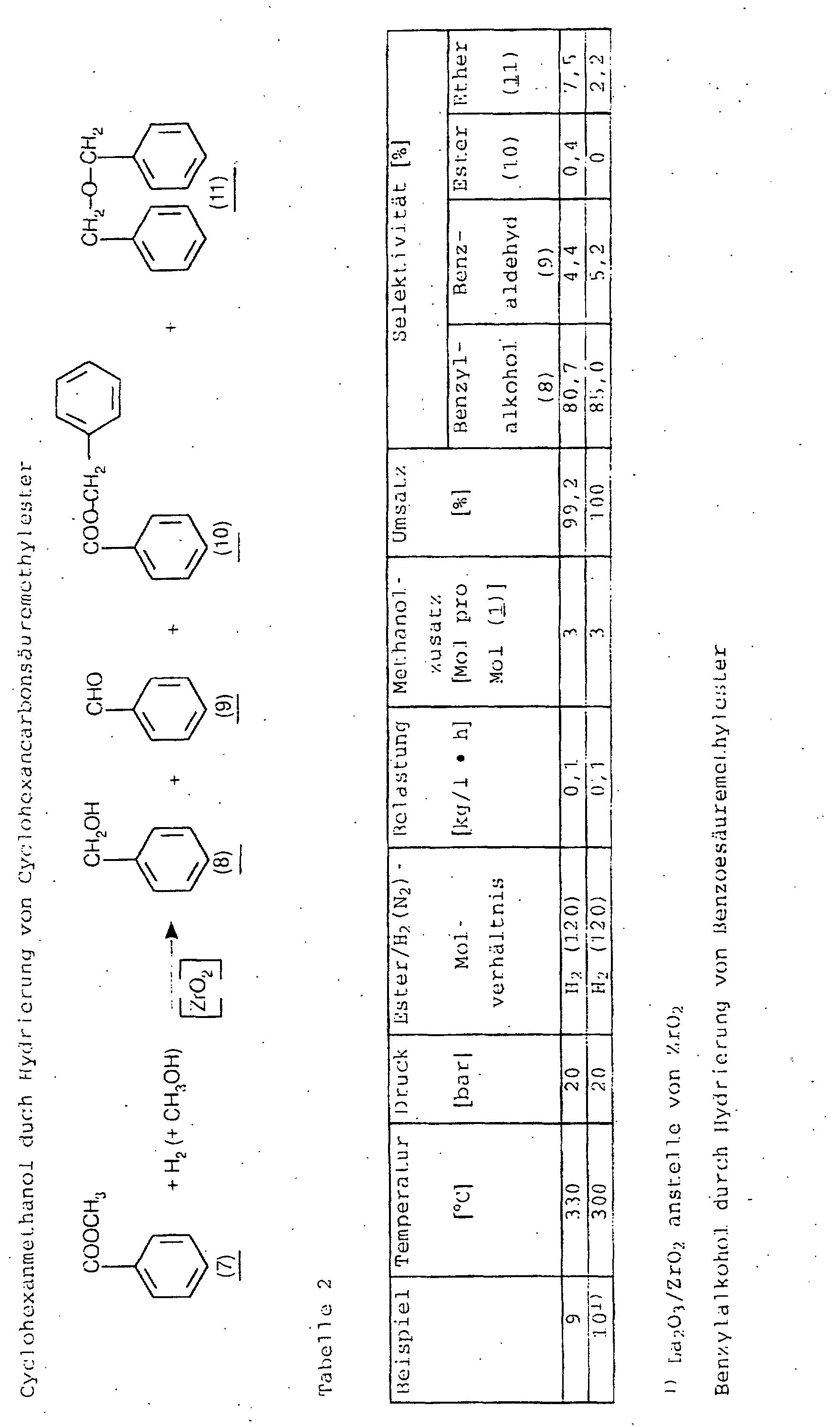

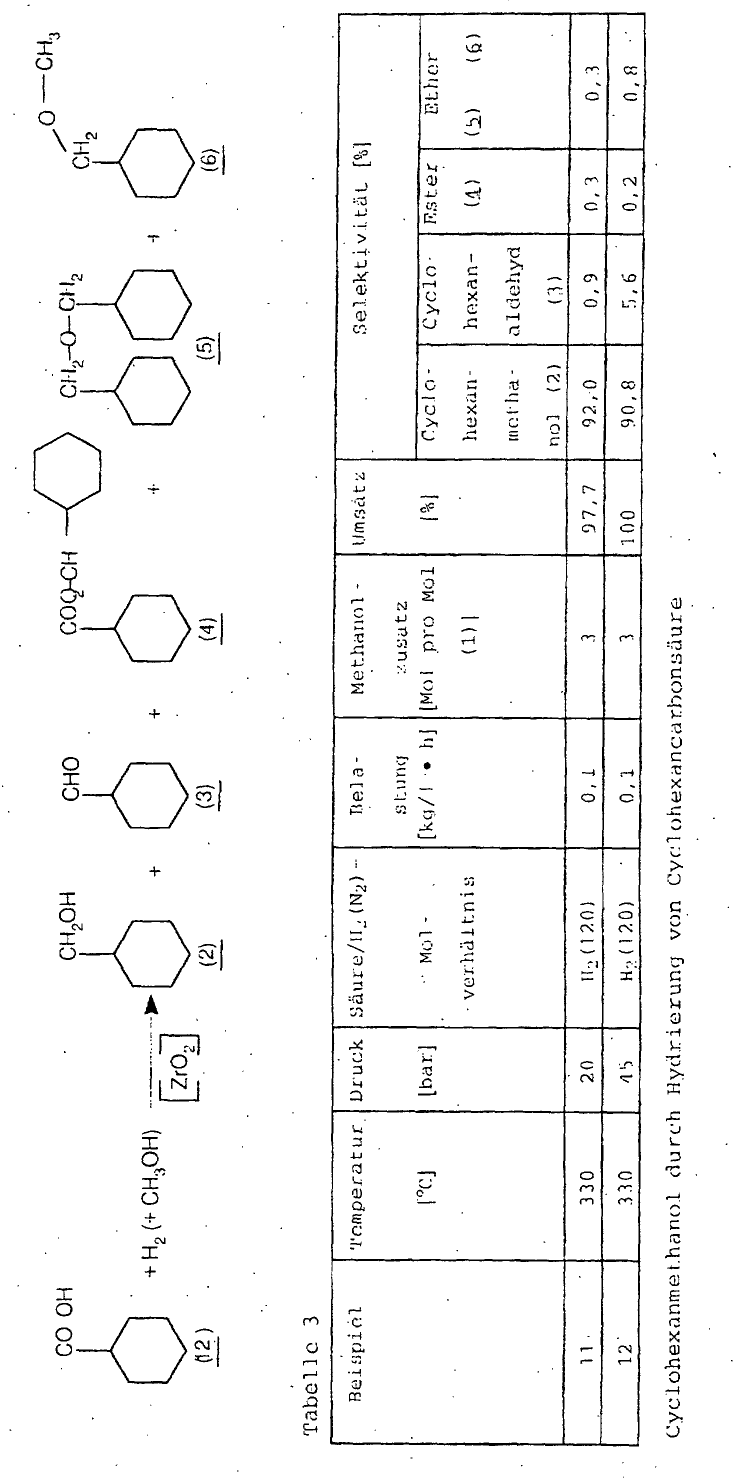

### Beispiel 13

Tetrahydropyran-4-carbonsäuremethylester wurde wie in Beispiel 6 beschrieben, aber bei 310°C/45 bar, hydriert. Die 4-Hydroxymethyltetrahydropyran-Selektivität betrug 76,4 % (Umsatz 97,4 %), die 4-Formyltetrahydropyran-Selektivität 2 %.

### Beispiel 14

n-Hexansäuremethylester wurde wie in Beispiel 6 beschrieben, aber bei 300°C/45 bar an einem Cr₂O₃/ZrO₂-Katalysator, hydriert. Die n-Hexanol-Ausbeute betrug 70 % (Umsatz 100 %), die n-Hexanal-Selektivität 0,5 %.

## Patentansprüche

1. Verfahren zur Herstellung von Alkoholen durch Gasphasenhydrierung von Carbonsäuren oder deren Estern bei erhöhter Temperatur und erhöhtem Druck in Gegenwart von Katalysatoren, die als hydrieraktive Bestandteile Oxide der vierten Hauptgruppe und/oder Nebengruppe enthalten oder aus diesen bestehen, **dadurch gekennzeichnet, daß** man die Hydrierung
a) bei Temperaturen von 250°C bis 400°C und Drücken von 5 bar bis 100 bar
b) unter Zusatz von primären oder sekundären Alkoholen durchführt, wobei das Molverhältnis Wasserstoff zu Alkohol maximal 800 und das Molverhältnis Wasserstoff zur eingesetzten Carbonsäure oder deren Ester 20 bis 300 beträgt.

2. Verfahren nach Anspruch 1, **dadurch gekennzeichnet, daß** das Molverhältnis Wasserstoff zu Alkohol 10 bis 300 beträgt.

3. Verfahren nach Anspruch 1, **dadurch gekennzeichnet, daß** man aliphatische primäre und/oder sekundäre Alkohole mit einem bis sieben Kohlenstoffatomen verwendet.

4. Verfahren nach Anspruch 1, **dadurch gekennzeichnet, daß** man Methanol als Alkohol verwendet.

5. Verfahren nach Anspruch 1, **dadurch gekennzeichnet, daß** man 0,5 bis 10 Mole Alkohol pro Mol Ausgangssäure oder Ester verwendet.

6. Verfahren nach Anspruch 1, **dadurch gekennzeichnet, daß** man Hydriertemperaturen von 300°C bis 350°C einhält.

7. Verfahren nach Anspruch 1, **dadurch gekennzeichnet, daß** man die Hydrierung bei Drucken von 25 bis 50 bar durchführt.

8. Verfahren nach Anspruch 1, **dadurch gekennzeichnet, daß** man monoklines, kubisches und/oder tetragonales Zirkondioxid verwendet.

9. Verfahren nach Anspruch 1, **dadurch gekennzeichnet, daß** man Zirkondioxid verwendet, das zu 50 bis 100 % aus monoklinem Zirkondioxid besteht.

10. Verfahren nach Anspruch 1, **dadurch gekennzeichnet, daß** der Oxidkatalysator eines oder mehrere Elemente der Lanthaniden, oder eines oder mehrere Elemente ausgewählt aus der Gruppe bestehend aus Chrom, Mangan, Zink, Indium, Zinn, Blei, Vanadium und Eisen enthält.

## Claims

1. A process for preparing alcohols by gas phase hydrogenation of carboxylic acids or esters thereof at elevated temperature and elevated pressure in the presence of a catalyst consisting of or comprising, as hydrogenating components, oxides of main group IV and/or subgroup IV, which comprises hydrogenating
a) at from 250°C to 400°C and from 5 bar to 100 bar
b) with the addition of primary or secondary alcohols, the molar hydrogen/alcohol ratio being at most 800 and the molar ratio of hydrogen to carboxylic acid or ester used being from 20 to 300.

2. A process as claimed in claim 1, wherein the molar hydrogen/alcohol ratio is from 10 to 300.

3. A process as claimed in claim 1, wherein aliphatic primary and/or secondary alcohols having 1 to 7 carbon atoms are used.

4. A process as claimed in claim 1, wherein the alcohol used is methanol.

5. A process as claimed in claim 1, wherein from 0.5 to 10 mol of alcohol are used per mole of starting acid or ester.

6. A process as claimed in claim 1, wherein the hydrogenation temperature is in the range from 300°C to 350°C.

7. A process as claimed in claim 1, wherein the hydrogenation pressure is in the range from 25 to 50 bar.

8. A process as claimed in claim 1, wherein monoclinic, cubic and/or tetragonal zirconium dioxide is used.

9. A process as claimed in claim 1, wherein the zirconium dioxide used comprises from 50 to 100% of monoclinic zirconium dioxide.

10. A process as claimed in claim 1, wherein the oxide catalyst comprises one or more lanthanide elements or one or more elements selected from the group consisting of chromium, manganese, zinc, indium, tin, lead, vanadium and iron.

## Revendications

1. Procédé pour la préparation d'alcools au moyen d'une hydrogénation en phase gazeuse d'acides carboxyliques ou de leurs esters, à une température élevée et sous une pression élevée, en présence de catalyseurs contenant, en tant que composants à activité d'hydrogénation, des oxydes du quatrième groupe principal et/ou sous-groupe, ou bien des catalyseurs qui sont composés de ces oxydes, **caractérisé en ce que** l'on réalise l'hydrogénation
a) à des températures allant de 250°C à 400°C et sous des pressions allant de 5 bars à 100 bars,
b) en ajoutant des alcools primaires ou secondaires, le rapport molaire de l'hydrogène à l'alcool étant de 800 au maximum, et le rapport molaire de l'hydrogène à l'acide carboxylique ou son ester mis en oeuvre étant de 20 à 300.

2. Procédé selon la revendication 1, **caractérisé en ce que** le rapport molaire de l'hydrogène à l'alcool est de 10 à 300.

3. Procédé selon la revendication 1, **caractérisé en ce que** l'on met en oeuvre les alcools aliphatiques primaires et/ou secondaires possédant un à sept atomes de carbone.

4. Procédé selon la revendication 1, **caractérisé en ce que** l'on met en oeuvre, en tant qu'alcool, le méthanol.

5. Procédé selon la revendication 1, **caractérisé en ce que** l'on met en oeuvre 0,5 mole à 10 moles d'alcool par mole d'ester ou d'acide de départ.

6. Procédé selon la revendication 1, **caractérisé en ce que** l'on réalise l'hydrogénation à des températures allant de 300°C à 350°C.

7. Procédé selon la revendication 1, **caractérisé en ce que** l'on réalise l'hydrogénation sous l'hydrogénation allant de 25 bars à 50 bars.

8. Procédé selon la revendication 1, **caractérisé en ce que** l'on met en oeuvre le dioxyde de zirconium monoclinique, cubique et/ou tétragonal.

9. Procédé selon la revendication 1, **caractérisé en ce que** l'on met en oeuvre du dioxyde de zirconium composé de 50% à 100% de dioxyde de zirconium monoclinique.

10. Procédé selon la revendication 1, **caractérisé en ce que** le catalyseur d'oxyde contient un ou plusieurs éléments des lanthanides ou un ou plusieurs éléments choisis dans le groupe formé par le chrome, le manganèse, le zinc, l'indium, l'étain, le plomb, le vanadium et le fer.
